## Europäisches Patentamt

## European Patent Office

## Office européen des brevets

(19)

(11) Veröffentlichungsnummer: **0 142 093**
**B1**

(12)

# EUROPÄISCHE PATENTSCHRIFT

(45) Veröffentlichungstag der Patentschrift:
24.08.88

(21) Anmeldenummer: **84113004.0**

(22) Anmeldetag: **29.10.84**

(51) Int. Cl.⁴: **C 07 D 241/52,** A 23 K 1/16,
A 61 K 31/495

(54) Staubfreie Chinoxalin-1,4-di-N-oxid enthaltende Zubereitung.

(30) Priorität: **11.11.83 DE 3340931**

(43) Veröffentlichungstag der Anmeldung:
**22.05.85 Patentblatt 85/21**

(45) Bekanntmachung des Hinweises auf die Patenterteilung:
**24.08.88 Patentblatt 88/34**

(84) Benannte Vertragsstaaten:
**AT BE CH DE FR GB IT LI LU NL SE**

(56) Entgegenhaltungen:
**EP - A - 0 073 390**
**EP - A - 0 134 444**
**DE - A - 1 670 935**

(73) Patentinhaber: **BAYER AG, Konzernverwaltung RP**
**Patentabteilung, D-5090 Leverkusen 1 Bayerwerk (DE)**

(72) Erfinder: **Hammen, Hans Werner, Dr., Ginsterweg 14,**
**D-5650 Solingen 1 (DE)**
Erfinder: **Voege, Herbert, Dr., Martin-Buber-Strasse 41,**
**D-5090 Leverkusen 3 (DE)**

## Beschreibung

Die Erfindung betrifft staubfreie Zubereitungen die

3-[N-(2-hydroxyethyl)-carbamoyl]-2-methyl-chinoxalin-1,4-di-N-oxid

enthalten, seine Herstellung und seine Verwendung zur Herstellung von Premixen und Futterzusatzmitteln in der Tieraufzucht.

Chinoxalin-1,4-di-N-oxide, insbesondere

2-Methyl-3-carbonsäureamidochinoxalin-1,4-di-N-oxide und

3-[N-(2-hydroxyethyl)-carbamoyl]-2-methyl-chinoxalin-1,4-di-N-oxid

und ihre Herstellungsverfahren sind aus der DE-C-1 670 935 bekannt. Diese Verbindungen weisen wertvolle chemotherapeutische und wachstumsfördernde Wirkungen in der Tieraufzucht auf (EP-A-73 390). Gemäss EP-A-134 444 (nicht vorveröffentlichter Stand der Technik) wird

3-[N-(2-hydroxyethyl)-carbamoyl]-2-methyl-chinoxalin-1,4-di-N-oxid

in bestimmter Korngrösse hergestellt um die Stabilität des Wirkstoffs zu steigern.

3-[N-(2-hydroxyethyl)-carbamoyl]-2-methyl-chinoxalin-1,4-di-N-oxid

fällt in den Herstellungsprozessen als fester kristalliner oder amorpher Stoff an, der zum Stäuben neigt. Wirkstoffe in Form von Staub oder Wirkstoffe, die eine Neigung zum Stäuben zeigen, verursachen Schwierigkeiten bei der Herstellung von Premixen oder Endformulierungen z.B. Endfuttern für die Tieraufzucht.

Es ist ausserdem nicht erwünscht, Produkte abzumischen die Wirkstoff-Staub enthalten, der eingeatmet werden kann, selbst wenn eine gesundheitliche Gefahr nicht sofort abzusehen ist.

Staubfreie Chinoxalin-1,4-di-N-oxide sind auch aus sicherheitstechnischen Gründen interessant. Chinoxalin-1,4-di-N-oxide neigen zum Verpuffen oder Explodieren. Sie brennen auch unter Ausschluss von Luft ab.

Solche Gefahr wird durch Abwesenheit von Staub, insbesondere auch in den Premixen reduziert. Der Zusatz von Wasser verhindert aber auch in der Reinsubstanz das Ausbreiten einer Explosion, da durch Verdampfen des Wassers die zur Ausbreitung benötigte Energie absorbiert wird. Ein solches nicht mehr explosives Produkt kann dann z.B. auch mit Luftfracht transportiert werden.

Gegenstand der Erfindung ist ein Verfahren zur Herstellung einer staubfreies

3-[N-(2-hydroxyethyl)-carbamoyl]-2-methyl-chinoxalin-1,4-di-N-oxid enthaltenden Zubereitung, das darin besteht, dass man

a) das nach üblichen Verfahren hergestellte und kristallin oder amorph aus einer Wasser enthaltenden Reaktionsmischung abgeschiedene filterfeuchte

3-[N-(2-hydroxyethyl)-carbamoyl]-2-methyl-chinoxalin-1,4-di-N-oxid

so trocknet, dass sein Wassergehalt 5–15 Gew.-% beträgt, oder für den Fall, dass das

3-[N-(2-hydroxyethyl)-carbamoyl]-2-methyl-chinoxalin-1,4-di-N-oxid

in einem wasserfreien oder nahezu wasserfreien System hergestellt wurde, diesem nach der Isolierung aus dem Reaktionsgemisch Wasser in einer Menge zusetzt, dass sein Wassergehalt 5–15 Gew.-% beträgt und

b) diesem wasserfeuchten

3-[N-(2-hydroxyethyl)-carbamoyl]-2-methyl-chinoxalin-1,4-di-N-oxid

5–15 Gew.% eines Fliessregulierungsmittels zusetzt.

Die Abmischung mit einem Fliessregulierungsmittel kann sowohl im Batch-Verfahren als auch kontinuierlich beim Austragen erfolgen. Es kann auch ein Mahlprozess mit dem Mischen verbunden werden.

Da das staubfreie

3-[N-(2-hydroxyethyl)-carbamoyl]-2-methyl-chinoxalin-1,4-di-N-oxid

mit der angegebenen Menge Wasser im Lauf der Lagerzeit Klumpen bilden könnte, muss dem Wirkstoff ein anderer Hilfsstoff zugesetzt werden, der dieses verhindert und die Wirkstoff-Kristalle oder -Partikel locker und getrennt hält. Sonst ist die Herstellung einer homogenen Vermischung oder eines Endfutters nur unter Einsatz eines zusätzlichen Mahlprozesses möglich.

Als Fliessregulierungsmittel können solche Substanzen eingesetzt werden, die Wasser zu einem gewissen Teil binden können. Als bekannteste Substanzen sind hier kolloidale und gefällte Kieselsäure zu nennen, aber auch Kieselgur, leichtes Calciumphosphat oder Calciumsulfat, das durch spezifische Verfahren Wasser adsorbierend hergestellt wurde, natürliche und synthetische Zeolithe, z.B. Bentonite, aber auch Tone u.a. Von organischen Fliessregulierungsmitteln sind besonders quervernetzte Polymere zu nennen, die grosse Wassermengen unter Quellen aufzunehmen vermögen. Diese können aus allen Bereichen der Polymerchemie kommen, z.B. quervernetzte Acrylate, Methacrylate, Polyamide, Polyvinylpyrrolidone, Cellulose-Derivate u.a. Naturprodukte, wie z.B. Stärken können ebenfalls von Interesse sein. Zusätzlich können Konservierungsstoffe wie z.B. Ester der p-Hydroxy-benzoesäure oder Propionsäure, Sorbinsäure oder deren Salze zugesetzt werden.

Weiterer Gegenstand der Erfindung ist die Verwendung der das

3-[N-(2-hydroxyethyl)-carbamyl]-2-methyl-chinoxalin-1,4-di-N-oxid

enthaltenden Zubereitung zur Herstellung von Futtermitteln für die Tieraufzucht.

Die beanspruchte Zubereitung kann in allen Bereichen der Tierzucht als Mittel zur Förderung und Beschleunigung des Wachstums und zur Verbesserung der Futterverwertung bei gesunden und kranken Tieren verwendet werden.

Die Wirksamkeit des

3-[N-(2-hydroxyethyl)-carbamoyl]-2-methyl-chinoxalin-1,4-di-N-oxids

ist hierbei weitgehend unabhängig von der Art und dem Geschlecht der Tiere. Besonders wertvoll erweist es sich bei der Aufzucht und Haltung von Jung- und Masttieren.

Als Tiere, bei denen die 3-[N-(2-hydroxyethyl)-carbamoyl]-2-methyl-chinoxalin-1,4-di-N-oxid enthaltende Zubereitung zur Förderung und Beschleunigung des Wachstums und zur Verbesserung der Futterverwertung eingesetzt werden können, seien beispielsweise folgende Nutz- und Ziertiere genannt: Warmblüter wie Rinder, Schweine, Pferde, Schafe, Ziegen, Katzen, Hunde, Kaninchen, Pelztiere, z.B. Nerze und Chinchilla, Geflügel, z.B. Hühner, Gänse, Enten, Truthähne, Tauben, Papageien und Kanarienvögel und Kaltblüter wie Fische, z.B. Karpfen und Reptilien, z.B. Schlangen.

Die Mengen 3-[N-(2-hydroxyethyl)-carbamoyl]-2-methyl-chinoxalin-1,4-di-N-oxid, die den Tieren zur Erreichung des gewünschten Effektes verabreicht werden, können wegen der günstigen Eigenschaften der Wirkstoffe weitgehend variiert werden. Sie liegt vorzugsweise bei 0,01 bis 50, insbesondere 0,1 bis 10 mg/kg Körpergewicht pro Tag. Die Dauer der Verabreichung kann von wenigen Stunden oder Tagen bis zu mehreren Jahren betragen. Die passende Menge 3-[N-(2-hydroxyethyl)-carbamoyl]-2-methyl-chinoxalin-1,4-di-N-oxid sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die das 3-[N-(2-hydroxyethyl)-carbamoyl]-2-methyl-chinoxalin-1,4-di-N-oxid enthaltende Zubereitung wird den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmässigen oder unregelmässigen Abständen oral oder parenteral erfolgen.

Aus Zweckmässigkeitsgründen ist in den meisten Fällen eine orale Verabreichung, insbesondere im Rhythmus der Nahrungs- und/oder Getränkeaufnahme der Tiere, vorzuziehen.

Die das Chinoxalin-1,4-di-N-oxid enthaltende Zubereitung kann reine Stoffmischung oder in formulierter Form, also in Mischung mit nicht-toxischen inerten Trägerstoffen beliebiger Art, z.B. mit Trägerstoffen und in Formulierungen, wie sie bei nutritiven Zubereitungen üblich sind, verabreicht werden.

Die das Chinoxalin-1,4-di-N-oxid enthaltende Zubereitung wird gegebenenfalls in formulierter Form zusammen mit pharmazeutischen Wirkstoffen, Mineralsalzen, Spurenelementen, Vitaminen, Eiweissstoffen, fetten, Farbstoffen und/oder Geschmacksstoffen in geeigneter Form verabreicht.

Empfehlenswert ist die orale Verabreichung zusammen mit dem Futter und/oder Trinkwasser, wobei je nach Bedarf der Chinoxalin-1,4-di-N-oxide der Gesamtmenge oder nur Teilen des Futters und/oder des Trinkwassers zugegeben wird.

Die das Chinoxalin-1,4-di-N-oxid enthaltende Zubereitung wird nach üblichen Methoden durch einfaches Mischen als reine Stoffmischung, vorzugsweise in fein verteilter Form oder in formulierter Form in Mischung mit essbaren nicht-toxischen Trägerstoffen, gegebenenfalls in Form von Premixen, oder eines Futterkonzentrates, dem Futter und/oder Trinkwasser beigefügt.

Das Futter und/oder Trinkwasser kann beispielsweise das Chinoxalin-1,4-di-N-oxid in einer Gewichtskonzentration von 5–500 ppm, insbesondere 10–300 ppm, enthalten. Die optimale Höhe der Konzentration Chinoxalin-1,4-di-N-oxide in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen oder speziellen Futterzusammensetzungen verwendet werden, die vorzugsweise das übliche, für eine ausgewogene Ernährung notwendige Gleichgewicht aus Energie- und Aufbaustoffen einschliesslich Vitaminen und Mineralstoffen enthalten. Das Futter kann sich beispielsweise zusammensetzen aus pflanzlichen Stoffen, z.B. Heu, Rüben, Getreide, Getreidenebenprodukten, tierischen Stoffen, z.B. Fleisch, Fetten, Knochenmehl, Fischprodukten, Vitaminen, z.B. Vitamin A, D-Komplex und B-Komplex, Proteinen, Aminosäuren, z.B. DL-Methionin und anorganischen Stoffen, z.B. Kalk und Kochsalz.

Futterkonzentrate enthalten das Chinoxalin-1,4-di-N-oxid neben essbaren Stoffen, z.B. Roggenmehl, Maismehl, Sojabohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Beispiel

In einen Mischer werden 910 kg 3-[N-(2-Hydroxyethyl)-carboamoyl]-2-methyl-chinoxalin-1,4-di-N-oxid mit einer Restfeuchte von 10% (= 819 kg Wirkstoff 100% + 91 kg Wasser) und 90 kg Kieselsäure eingefüllt und homogen gemischt. Die fliessfähige Mischung wird in Gebinde abgefüllt. Sie enthält 81,9% Wirkstoff, 9,1% Wasser und 9% Kieselsäure.

Nachweis der Sicherheit gegenüber reinem Wirkstoff.

In zwei Schalen wird hinter Sicherheitsglas im ersten Versuch die Reinsubstanz, im zweiten Versuch die Mischung nach obigem Herstellungsbeispiel mit einem glühenden Draht berührt. Im ersten Versuch verpufft die gesamte Substanzmenge unter schwarzer Rauchentwicklung.

Im zweiten Versuch entzündet sich die Substanz am Berührungspunkt, die Verpuffung breitet sich aber nicht weiter aus und erlischt sofort wieder.

**Patentansprüche**

1. Verfahren zur Herstellung einer staubfreies 3-[N-(2-hydroxyethyl)-carbamoyl]-2-methyl-chinoxalin-1,4-di-N-oxid enthaltenden Zubereitung, dadurch gekennzeichnet, dass man

a) das nach üblichen Verfahren hergestellte und kristallin oder amorph aus einer Wasser enthaltenden Reaktionsmischung abgeschiedene filterfeuchte 3-[N-(2-hydroxyethyl)-carbamoyl]-2-methyl-chinoxalin-1,4-di-N-oxid so trocknet, dass sein Wassergehalt 5–15 Gew.-% beträgt, oder für den Fall, dass das 3-[N-(2-hydroxyethyl)-carbamoyl]-2-methyl-chinoxalin-1,4-di-N-oxid in einem wasserfreien oder nahezu wasserfreiem System hergestellt wurde, diesem nach der Isolierung aus dem Reaktionsgemisch Wasser in einer Menge zusetzt, dass der Wassergehalt 5–15 Gew.-% beträgt und

b) diesem wasserfeuchten 3-[N-(2-hydroxyethyl)-carbamoyl]-2-methyl-chinoxalin-1,4-di-N-oxid 5–15 Gew.-% eines Fliessregulierungsmittels zusetzt.

2. Verfahren nach Anspruch 1, dadurch gekennzeichnet, dass man ein Fliessregulierungsmittel auf Kieselsäurebasis zusetzt.

3. Zubereitung welche neben 3-[N-(2-hydroxyethyl)-carbamoyl]-2-methyl-chinoxalin-1,4-di-N-oxid 5–15 Gew.-% Wasser und 5–15 Gew.-% eines Fliessregulierungsmittels enthält.

4. Zubereitung welche neben 3-[N-(2-hydroxyethyl)-carbamoyl]-2-methyl-chinoxalin-1,4-di-N-oxid 5–15 Gew.-% Wasser, 5–15 Gew.-% eines Fliessregulierungsmittels und ein Konservierungsmittel enthält.

5. Verwendung der Zubereitung gemäss Anspruch 3 zur Herstellung von Futterzusatzmitteln oder Futtermitteln für Tiere.

**Claims**

1. Process for the preparation of a formulation containing dust-free 3-[N-(2-hydroxyethyl)carbamoyl]-2-methylquionoxaline 1,4-di-N-oxide, characterized in that

a) the 3-[N-(2-hydroxyethyl)carbamoyl]-2-methylquinoxaline 1,4-di-N-oxide, which has been prepared by customary processes and precipitated out, crystalline or amorphous, from a reaction mixture containing water and is moist from the filter, is dried in such a manner that its water content is 5–15% by weitht, or, in the case where the 3-[N-(2-hydroxyethyl)carbamoyl]-2-methylquinoxaline 1,4-di-N-oxide has been prepared in an anhydrous or virtually anhydrous system, after isolation of the latter from the reaction mixture, water is added to it in an amount, such that the water content is 5–15% by weight, and

b) 5–15% by weight of a flow-regulating agent is added to this 3-[N-(2-hydroxyethyl)carbamoyl]-2-methylquinoxaline 1,4-di-N-oxide, which is moist with water.

2. Process according to Claim 1, characterized in that a flow-regulating agent based on silica is added.

3. Formulation which, in addition to 3-[N-(2-hydroxyethyl)carbamoyl]-2-methylquinoxaline 1,4-di-N-oxide, contains 5–15% by weight of water and 5–15% by weight of a flow-regulating agent.

4. Formulation which, in addition to 2-[N-(2-hydroxyethyl)carbamoyl]-3-methylquinoxaline 1,4-di-N-oxide, contains 5–15% by weight of water, 5–15% by weight of a flow-regulating agent and a preservative.

5. Use of the formulation according to Claim 3 for the preparation of feed additives or feedstuffs for livestock.

**Revendications**

1. Procédé de production d'une préparation contenant le 1,4-di-N-oxyde de 3-[N-(2-hydroxyéthyl)-carbamoyl]-2-méthyl-quinoxaline exempt de poussière, caractérisé par les étapes suivantes:

a) séchage du 1,4-di-N-oxyde de 3-[N-(2-hydroxyéthyl)-carbamoyl]-2-méthyl-quinoxaline humide après filtration séparé à l'état amorphe ou cristallin d'un mélange réactionnel contenant de l'eau et obtenu par un procédé de fabrication usuel, ce séchage étant tel que la teneur en eau soit de 5 à 15% en poids ou bien, au cas où le 1,4-di-N-oxyde de 3-[-(2-hydroxyéthyl)-carbamoyl]-2-méthyl-quinoxaline a été produit dans un système anhydre ou quasi-anhydre, l'addition à celui-ci, après sa séparation du mélange réactionnel, d'eau en quantité telle que la teneur en eau soit de 5 à 15% en poids, et

b) l'addition à ce 1,4-di-N-oxyde de 3-[N-(2-hydroxyéthyl)-carbamoyl]-2-méthyl-quinoxaline humide de 5 à 15% en poids d'un agent régularisant l'écoulement.

2. Procédé selon la revendication 1, caractérisé en ce que l'on ajoute un agent régularisant l'écoulement à base d'acide silicique.

3. Préparation contenant, outre le 1,4-di-N-oxyde de 3[N-(2-hydroxyéthyl)-carbamoyl]-2-méthyl-quinoxaline, 5 à 15% en poids d'eau et 5 à 15% en poids d'un agent régularisant l'écoulement.

4. Préparation contenant, outre le 1,4-di-N-oxyde de 3-[N-(2-hydroxyéthyl)-carbamoyl]-2-méthyl-quinoxaline,

5 à 15% en poids d'eau, 5 à 15% en poids d'un agent régularisant l'écoulement ainsi qu'un agent de conservation.

5. Utilisation de la préparation selon la revendication 3, pour préparer des additifs pour aliments pour bétail ou bien des aliments pour bétail.